(19) Europäisches Patentamt
European Patent Office
Office européen des brevets

(11) **EP 3 698 800 A1**

(12) **EUROPEAN PATENT APPLICATION**
published in accordance with Art. 153(4) EPC

(43) Date of publication:
26.08.2020 Bulletin 2020/35

(51) Int Cl.:
*A61K 33/00* (2006.01)    *A61K 9/70* (2006.01)
*A61K 47/32* (2006.01)    *A61M 35/00* (2006.01)
*A61P 9/08* (2006.01)    *A61P 17/00* (2006.01)

(21) Application number: 18868742.0

(22) Date of filing: 17.10.2018

(86) International application number:
PCT/JP2018/038594

(87) International publication number:
WO 2019/078232 (25.04.2019 Gazette 2019/17)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 20.10.2017 JP 2017203929

(71) Applicants:
• Mitsui Chemicals, Inc.
Minato-ku
Tokyo 105-7122 (JP)
• Kao Corporation
Chuo-ku
Tokyo 103-8210 (JP)

(72) Inventors:
• MATAYOSHI, Tomoya
Sodegaura-shi
Chiba 299-0265 (JP)
• WATANABE, Takayuki
Sodegaura-shi
Chiba 299-0265 (JP)
• ERIGUCHI, Michio
Sodegaura-shi
Chiba 299-0265 (JP)
• TANAKA, Masahito
Tokyo 1318501 (JP)

(74) Representative: Mewburn Ellis LLP
Aurora Building
Counterslip
Bristol BS1 6BX (GB)

(54) **CARBON DIOXIDE SUSTAINED RELEASE PACK FOR SKIN APPLICATIONS, AND METHOD FOR SUSTAINABLY RELEASING CARBON DIOXIDE ONTO SKIN**

(57) A carbon dioxide slow-release pack for skin according to the present invention is a carbon dioxide slow-release pack which has a bag shape for slowly releasing carbon dioxide enclosed therein, thereby bringing the carbon dioxide into contact with the skin, and is formed into a bag shape by at least a resin sheet (A), and the resin sheet (A) satisfies at least the following requirements (A-1) and (A-2).

(A-1) A carbon dioxide permeability measured under the conditions of 23°C and a relative humidity of 0% according to JIS K7126-1 is 20 L/(m$^2$·day·atm) or more.

(A-2) An Oken-type air permeability measured at 23°C according to JIS P8117: 2009 is less than 5 $\mu$m/(Pa·s).

FIG. 2

HEAT SEALING REGION
OPENING (EYE PORTIONS, NOSE PORTION, AND MOUTH PORTION)
INTERNAL BUFFER MATERIAL PORTION SURROUNDED BY RESIN SHEETS (A) AND (B)

C-C' CROSS-SECTION

EP 3 698 800 A1

**Description**

TECHNICAL FIELD

The present invention relates to a carbon dioxide slow-release pack for skin and a method of slowly releasing carbon dioxide to the skin.

BACKGROUND ART

There have been known methods of using a physiologically active action of carbon dioxide through the percutaneous absorption of the carbon dioxide from outside the human body to skin. Among methods of introducing carbon dioxide to the skin, techniques in which a state in which carbon dioxide is sealed in a gaseous state or a carbon dioxide-containing aqueous solution in which carbon dioxide is dissolved (hereinafter, also simply referred to as carbonated water) is sealed in a bag is kept, and the carbon dioxide gas or carbonated water is slowly released from a surface of the bag to be brought into contact with the skin have been known.

For example, Patent Document 1 (Japanese Unexamined Patent Publication No. 2002-326938) discloses a material for the percutaneous and transmucous absorption of carbon dioxide which is provided with a gas-permeable material having a carbon dioxide permeability of 1,000 to 100,000 $cm^2/m^2 \cdot day \cdot atm$ between a carbon dioxide supplying portion and an affected part in order to enable early recovery of a skin or mucous membrane damage by a peripheral vessel dilation action of a skin or a mucous membrane based on the medicinal effect of the carbon dioxide. In Examples in Patent Document 1, a polystyrene film (of an unknown thickness), a low-density polyethylene wrap film (of a thickness of 10 $\mu$m), a polyurethane film (of an unknown thickness), and a poly-4-methylpentene-1 film (of a thickness of 40 $\mu$m) are used as the gas-permeable material. In addition, it is described that a gas-permeable material may be used for a bag-shaped container to store a carbon dioxide supply source such as carbonated water.

As a method of qualitatively and easily evaluating a blood circulation promoting effect accompanied by the percutaneous absorption of carbon dioxide into the skin, a method of determining whether the skin is flushed after supply of the carbon dioxide to the skin for a predetermined period of time has been known (for example, Patent Document 2: Japanese Unexamined Patent Publication No. 2005-225832) .

In addition, Non-Patent Document 1 reports that flushing is recognized as a skin quality improving effect of carbon dioxide in a case where the skin quality is improved.

RELATED DOCUMENT

PATENT DOCUMENT

[Patent Document 1] Japanese Unexamined Patent Publication No. 2002-326938
[Patent Document 2] Japanese Unexamined Patent Publication No. 2005-225832

NON-PATENT DOCUMENT

[Non-Patent Document 1] Jiro Kadomoto (2015), "blending of carbon dioxide with cosmetics and skin quality improving effect", Fragrance Journal (43) (7), p. 40 to 44

SUMMARY OF THE INVENTION

TECHNICAL PROBLEM

As described above, there has been known a method of slowly releasing carbon dioxide to the skin using a bag-shaped container with carbon dioxide sealed inside to continuously supply the carbon dioxide to the skin, thereby promoting blood circulation of the skin.

Here, according to the study of the present inventors, it has been found that there is room for improvement in the bag-shaped container described in Patent Document 1 in obtaining a sufficient blood circulation promoting effect and in continuously releasing the carbon dioxide and maintaining the blood circulation promoting effect for a long period of time.

The present invention has been contrived in view of the above circumstances, and provides a carbon dioxide slow-release pack for skin which has an excellent blood circulation promoting effect and can continuously maintain the blood circulation promoting effect for a long period of time.

SOLUTION TO PROBLEM

The present inventors have conducted intensive studies to solve the above problems. As a result, the present inventors have obtained knowledge that a carbon dioxide slow-release pack using a resin sheet whose carbon dioxide permeability and Oken-type air permeability are within a specific range has an excellent blood circulation promoting effect and can maintain the blood circulation promoting effect for a long period of time, and completed the present invention.

That is, according to the present invention, provided are the following carbon dioxide slow-release pack for skin and a method of slowly releasing carbon dioxide to the skin.

[1] A carbon dioxide slow-release pack for skin which has a bag shape for slowly releasing carbon dioxide enclosed therein, thereby bringing the carbon dioxide into contact with the skin, the pack including:

a resin sheet (A), in which the carbon dioxide slow-release pack is formed into a bag shape by at least the resin sheet (A), and
the resin sheet (A) satisfies at least the following requirements (A-1) and (A-2).
(A-1) A carbon dioxide permeability measured under the conditions of 23°C and a relative humidity of 0% according to JIS K7126-1 is 20 L/(m$^2$·day·atm) or more.
(A-2) An Oken-type air permeability measured at 23°C according to JIS P8117: 2009 is less than 5 $\mu$m/(Pa·s).

[2] The carbon dioxide slow-release pack for skin according to [1],
in which the resin sheet (A) further satisfies the following requirement (A-3).
(A-3) Young's modulus E at 23°C is 10 MPa to 500 MPa.
[3] The carbon dioxide slow-release pack for skin according to [1] or [2],
in which the resin sheet (A) further satisfies the following requirement (A-4).
(A-4) tensile elongation at break at 23°C is 30% or more.
[4] The carbon dioxide slow-release pack for skin according to any one of [1] to [3], further including:

a resin sheet (B) satisfying the following requirements (B-1) and (B-2).
(B-1) Water resistance measured at 23°C according to JIS L1092A: 2009 is 350 mbar or more.
(B-2) An Oken-type air permeability measured at 23°C according to JIS P8117: 2009 is less than 5 $\mu$m/(Pa·s).

[5] The carbon dioxide slow-release pack for skin according to [4],
in which the resin sheet (A) is disposed on a skin side, and the resin sheet (B) is disposed on an atmosphere side.
[6] The carbon dioxide slow-release pack for skin according to any one of [1] to [5],
in which the resin sheet (A) includes a polyolefin film or sheet.
[7] The carbon dioxide slow-release pack for skin according to [6],
in which the polyolefin film or sheet includes at least one selected from polyethylene, an ethylene-based copolymer, a propylene-based copolymer, and a 4-methyl-1-pentene-based polymer.
[8] The carbon dioxide slow-release pack for skin according to [6] or [7],
in which the polyolefin film or sheet includes a microporous film or sheet.
[9] The carbon dioxide slow-release pack for skin according to [8],
in which the microporous film or sheet includes one or more selected from a foamed sheet formed of a 4-methyl-1-pentene-based polymer, a polypropylene-based microporous film, and a filler-containing polyolefin-based microporous film.
[10] The carbon dioxide slow-release pack for skin according to any one of [1] to [9],
in which the resin sheet (A) includes a copolymer having a constituent unit derived from 4-methyl-1-pentene and a constituent unit derived from an $\alpha$-olefin having 2 to 20 carbon atoms other than 4-methyl-1-pentene.
[11] The carbon dioxide slow-release pack for skin according to any one of [1] to [10], further including:
a fibrous reinforcing material on a surface of the resin sheet (A) or inside the resin sheet (A).
[12] The carbon dioxide slow-release pack for skin according to any one of [1] to [11],
in which the resin sheet (A) includes a layer formed of a 4-methyl-1-pentene-based polymer and a layer formed of a thermoplastic elastomer.
[13] The carbon dioxide slow-release pack for skin according to any one of [1] to [12], which is a pack for face.
[14] The carbon dioxide slow-release pack for skin according to any one of [1] to [13],
in which at least one selected from carbon dioxide and a carbon dioxide source is enclosed therein.

[15] A method of slowly releasing carbon dioxide to skin using the carbon dioxide slow-release pack for skin according to any one of [1] to [14], the method including:

bringing the resin sheet (A) into close contact with the skin, and slowly releasing the carbon dioxide from the inside of the carbon dioxide slow-release pack for skin between the resin sheet (A) and the skin to continuously supply the carbon dioxide to the skin.

ADVANTAGEOUS EFFECTS OF INVENTION

According to the present invention, it is possible to provide a carbon dioxide slow-release pack for skin which has an excellent blood circulation promoting effect and can continuously maintain the blood circulation promoting effect for a long period of time.

BRIEF DESCRIPTION OF THE DRAWINGS

The above-described object, other objects, characteristics, and advantages will become more apparent from preferable embodiments to be described below and the following accompanying drawings.
Fig. 1 is a plan view schematically showing an example of a structure of a carbon dioxide slow-release pack for skin according to an embodiment of the present invention.
Fig. 2 is a plan view schematically showing an example of the structure of the carbon dioxide slow-release pack for skin according to the embodiment of the present invention.
Fig. 3 is a perspective view schematically showing an example of a part of the structure of the carbon dioxide slow-release pack for skin according to the embodiment of the present invention.
Fig. 4 is a perspective view schematically showing an example of a part of the structure of the carbon dioxide slow-release pack for skin according to the embodiment of the present invention.

DESCRIPTION OF EMBODIMENTS

Hereinafter, embodiments of the present invention will be described with reference to the drawings. In all the drawings, similar constituent elements are denoted by common reference signs, and description thereof will not be repeated. "A to B" indicating a numerical value range represents equal to or more than A and equal to or less than B.
Resin sheets (A) and (B) according to the present embodiment include not only sheets but also films.

<Carbon Dioxide Slow-Release Pack for Skin>

A carbon dioxide slow-release pack for skin according to the present embodiment is a carbon dioxide slow-release pack having a bag shape for slowly releasing carbon dioxide enclosed therein, thereby bringing the carbon dioxide into contact with the skin, and is formed into a bag shape by using at least a resin sheet (A). The resin sheet (A) satisfies at least the following requirements (A-1) and (A-2).
(A-1) A carbon dioxide permeability measured under the conditions of 23°C and a relative humidity of 0% according to JIS K7126-1 is 20 $L/(m^2 \cdot day \cdot atm)$ or more.
(A-2) An Oken-type air permeability measured at 23°C according to JIS P8117: 2009 is less than 5 $\mu m/(Pa \cdot s)$.
Here, in a case where the carbon dioxide permeability of the resin sheet (A) is equal to or more than the lower limit value and the Oken-type air permeability of the resin sheet (A) is less than the upper limit value, the amount of the carbon dioxide continuously supplied to the skin is an appropriate value, and thus a blood circulation promoting effect can be improved and maintained for a long period of time. In a case where the Oken-type air permeability of the resin sheet (A) is less than the upper limit value, the amount of carbon dioxide supplied is not excessively large and is appropriate, and thus carbon dioxide can be continuously supplied for a long period of time.

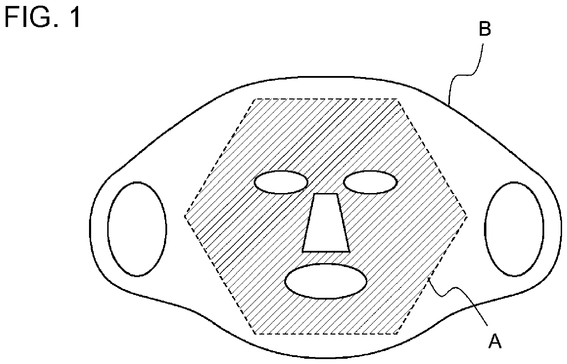

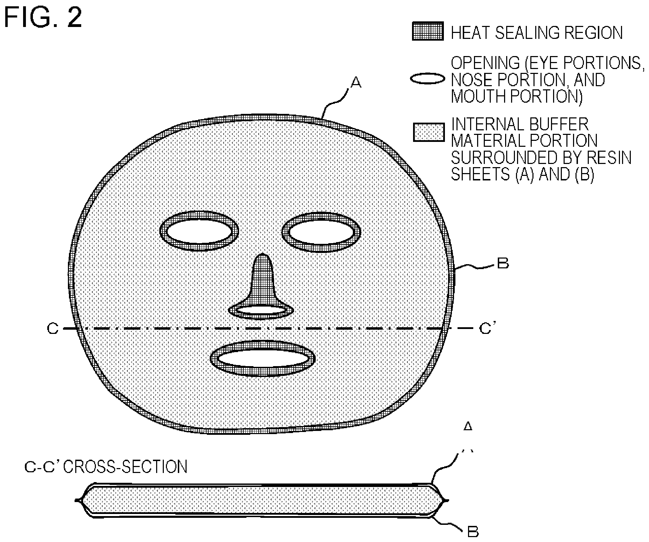

Figs. 1 and 2 are plan views schematically showing an example of a structure of the carbon dioxide slow-release pack for skin according to the embodiment of the present invention.
A part of a human body on which the carbon dioxide slow-release pack for skin according to the present embodiment is used is not particularly limited. For example, the carbon dioxide slow-release pack for skin can be used as a face pack in the form shown in Figs. 1 and 2.

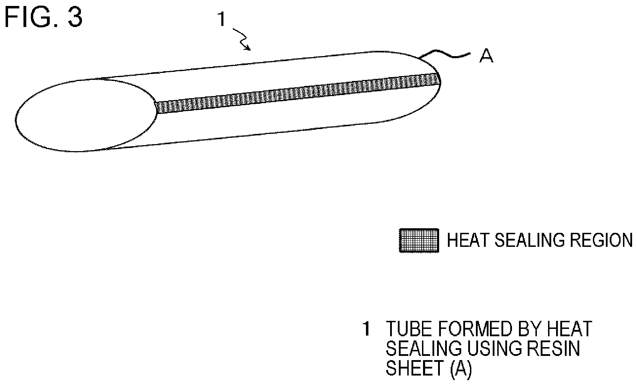

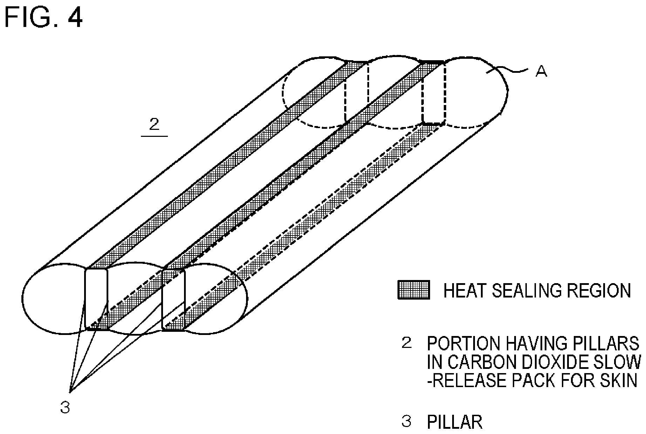

Figs. 3 and 4 are perspective views schematically showing an example of a part of the structure of the carbon dioxide slow-release pack for skin according to the embodiment of the present invention.
A part of a human body on which the carbon dioxide slow-release pack for skin according to the present embodiment is used is not particularly limited. For example, by employing the form shown in Fig. 3 or 4, the carbon dioxide slow-release pack for skin can be used as a pack for hand, arm, neck, shoulder, back, abdomen, leg, or the like as well as a face pack.

As described above, there has been known a method of slowly releasing carbon dioxide to the skin using a bag-shaped container with carbon dioxide sealed inside to continuously supply the carbon dioxide to the skin, thereby promoting blood circulation of the skin.

Here, according to the study of the present inventors, it has been found that there is room for improvement in the bag-shaped container described in Patent Document 1 in obtaining a sufficient blood circulation promoting effect and in continuously releasing the carbon dioxide and maintaining the blood circulation promoting effect for a long period of time.

Therefore, the present inventors have intensively studied to solve the problems. As a result, the present inventors have obtained knowledge that a carbon dioxide slow-release pack using a resin sheet whose carbon dioxide permeability and Oken-type air permeability are within a specific range has an excellent blood circulation promoting effect and can maintain the blood circulation promoting effect for a long period of time, and completed the present invention.

That is, according to the carbon dioxide slow-release pack for skin of the present embodiment, it is possible to obtain a good blood circulation promoting effect and to maintain the blood circulation promoting effect for a long period of time by using the resin sheet (A) satisfying at least the requirements (A-1) and (A-2).

As described above, according to the present embodiment, it is possible to realize a carbon dioxide slow-release pack for skin which has an excellent blood circulation promoting effect and can maintain the blood circulation promoting effect for a long period of time.

The carbon dioxide slow-release pack for skin according to the present embodiment is preferably further provided with a resin sheet (B) satisfying the following requirements (B-1) and (B-2).

(B-1) Water resistance measured at 23°C according to JIS L1092A: 2009 is 350 mbar or more.

(B-2) An Oken-type air permeability measured at 23°C according to JIS P8117: 2009 is less than 5 $\mu$m/(Pa·s).

In a case where the carbon dioxide slow-release pack for skin according to the present embodiment further includes the resin sheet (B), the resin sheet (A) can be disposed on the skin side, and the resin sheet (B) can be disposed on the atmosphere side. As a result, since the resin sheet (B) can suppress the leakage of carbon dioxide to the atmosphere, the carbon dioxide enclosed in the carbon dioxide slow-release pack for skin can be more effectively slowly released to the skin.

For example, the carbon dioxide slow-release pack for skin according to the present embodiment can be obtained by forming the resin sheet (A) and the optional resin sheet (B) into such a bag shape that at least one selected from carbon dioxide and a carbon dioxide source can be stored.

In a case where the resin sheet (A) and/or the resin sheet (B) have heat sealability, the resin sheets (A) and (B) may be directly heat sealed and formed into a bag shape. Otherwise, between the resin sheets (A) and (B), a heat sealing layer formed of a resin compatible with the resin sheets may be interposed and heat sealed for forming into a bag shape.

Alternatively, the resin sheets (A) and (B) can be formed into a bag shape by bonding the resin sheets with an adhesive.

In a case where a bag-shaped carbon dioxide slow-release pack for skin is formed by heat sealing or the like using the resin sheets (A) and (B), and a high-concentration carbon dioxide gas is introduced into the pack, a buffer material such as soft sponge foam, pulp, or nonwoven fabric may be previously inserted into the bag. In a case where a buffer material such as soft sponge foam is present inside the bag, the carbon dioxide slow-release pack for skin easily follows a shape of a human body surface when being brought into close contact with the human body surface, and the texture can be improved. In a case where the buffer material is soft sponge foam, for example, urethane, NBR, SBR, polyethylene, polypropylene, or the like can be used as the material.

From the viewpoint of light weight property and flexibility, the density of the soft sponge foam is preferably 0.05 g/cm$^3$ to 0.3 g/cm$^3$. From the viewpoint of securing shape conformability, the thickness of the soft sponge foam is preferably 1 mm to 30 mm. A sponge material which is frequently used as makeup puff can also be preferably used. For example, a carbon dioxide slow-release pack for skin intended for a facial part may be formed into a bag shape by heat sealing, in which as shown in the schematic view of Fig. 2, the resin sheet (A) is used as a sheet to be brought into contact with the face, the resin sheet (B) is used as a sheet on the opposite side, openings are partially provided only for eye portions, a nose portion, and a mouth portion, an outer shape is formed along a peripheral edge portion of the face, and soft sponge foam is incorporated therein.

In a case where vacuum moldability or cast moldability is provided, a technique for shaping one or both of the resin sheets (A) and (B) into a three-dimensional shape which is likely to follow the three-dimensional shape of a human body may be used for forming into a bag shape.

The carbon dioxide slow-release pack for skin according to the present embodiment may have a port portion through which a carbon dioxide gas or carbonated water is introduced. For example, when peripheral edge portions of the resin sheets (A) and (B) are heat sealed for forming into a bag shape, the port portion can be provided at a part of the peripheral edge portion.

The port portion may be a screw cap type, or may be mechanically sealed with a structure in which an opening is provided at one end portion of the carbon dioxide slow-release pack for skin and the resin sheet at the one end is held with a fitting-type clip.

For example, a structure may be provided in which as shown in Fig. 3, a tubular shape 1 is formed by heat sealing with the use of the resin sheet (A), and then can be sealed by holding with a screw cap or a fitting-type clip at both end portions thereof.

In addition, as a method of improving the deterioration in conformability to a human body surface due to the swelling of the carbon dioxide pack for skin containing a carbon dioxide gas or carbonated water, for example, a structure may be provided in which as shown in Fig. 4, a part 2 in the carbon dioxide pack for skin is provided with pillars 3 by heat sealing or the like to suppress the swelling and to improve the conformability to the shape of the human body surface.

The carbon dioxide slow-release pack for skin according to the present embodiment is preferably used in a state of being brought into close contact with a human body surface with water interposed therebetween. By bringing the carbon dioxide slow-release pack for skin into close contact with the skin, scattering of carbon dioxide to areas other than the skin can be suppressed, and the carbon dioxide can be efficiently supplied to the skin. Furthermore, moisture is preferably interposed between the carbon dioxide slow-release pack for skin and the skin since the supply of the gaseous carbon dioxide transmitted through the resin sheet (A) to the skin is promoted due to the moisture interposed between the carbon dioxide slow-release pack for skin and the skin even in a case where the resin sheet (A) does not have water permeability, and blood circulation is promoted. In a case where a human body surface of a part to which the carbon dioxide slow-release pack for skin is applied is in a moist state due to sweating or the environment, the carbon dioxide slow-release pack for skin is brought into close contact with the skin without the addition of water from the outside, whereby carbon dioxide can be efficiently supplied to the skin.

The amount of moisture interposed between the carbon dioxide slow-release pack for skin according to the present embodiment and the skin is not particularly limited as long as the skin surface is wet with water.

In addition, examples of the method of supplying moisture between the carbon dioxide slow-release pack for skin according to the present embodiment and the skin include: a method in which before the application of the carbon dioxide slow-release pack for skin to the skin, the skin is wet with moisture by performing spraying on the skin or wiping the skin with cloth containing water; a method in which a moisturizing skin lotion, cosmetic cream, cosmetic pack, gel, or the like containing moisture is applied to the skin; and a method in which a cotton-like sponge, nonwoven fabric, gauze, bandage, cosmetic mask, or the like formed of lint, cotton, pulp, polyester, polyurethane, polyamide, or the like having a small thickness (for example, thickness range: 0.05 mm to 0.5 mm) and excellent water absorbability, water permeability, and air permeability is allowed to contain moisture and the moisture is interposed between the carbon dioxide slow-release pack for skin and the skin.

The standard of the amount of moisture of the skin can be quantitatively determined using, for example, a skin moisture content measuring device using a bioelectrical impedance method. Examples of the skin moisture content measuring device include product name: BELULU skin checker (product number: KRD1042), manufactured by Beautiful Angel Inc. (Kireido). As a method of measuring the moisture content, for example, in a case where moisture is applied to the skin before the application of the carbon dioxide slow-release pack for skin to the skin, a moisture content X% measured immediately before the application of moisture and a moisture content Y% measured immediately after the application of moisture are measured with respect to a skin region to which the carbon dioxide slow-release pack for skin is pressed, and an increase Z% in moisture content before and after the application of moisture is obtained by the following expression.

$$Z = Y - X \ (\%)$$

In this case, before the application of the carbon dioxide slow-release pack for skin to the skin, the moisture is preferably applied such that the increase Z in moisture content before and after the application of moisture is 15% or more. The increase Z in moisture content is more preferably 20% to 30%.

The carbon dioxide slow-release pack for skin according to the present embodiment is used in a state in which at least one selected from carbon dioxide and a carbon dioxide source is enclosed therein.

The carbon dioxide slow-release pack for skin according to the present embodiment can continuously supply carbon dioxide to the skin by bringing the resin sheet (A) into close contact with the skin, and slowly releasing the carbon dioxide from the inside of the carbon dioxide slow-release pack for skin between the resin sheet (A) and the skin.

In the present embodiment, the method of introducing carbon dioxide into the carbon dioxide slow-release pack for skin is not particularly limited, and examples thereof include: a method of directly introducing an aqueous solution in which only carbon dioxide is dissolved at a high concentration into the carbon dioxide slow-release pack for skin; a method of generating carbon dioxide by mixing and reacting a carbonate, an acid, and water in the carbon dioxide slow-release pack for skin to prepare a carbon dioxide aqueous solution; a method of directly introducing gaseous carbon dioxide from the outside into the carbon dioxide slow-release pack for skin; and a method in which the sealed carbon dioxide slow-release pack for skin is put under a high-concentration carbon dioxide gas atmosphere or a high-concentration carbon dioxide aqueous solution atmosphere, and carbon dioxide is gradually diffused into the sealed carbon dioxide slow-release pack for skin with a concentration gradient of the carbon dioxide inside and outside the pack as a driving force to introduce the carbon dioxide into the carbon dioxide slow-release pack for skin (hereinafter, tentatively referred to as an inverse diffusion filling method).

Among these, the method in which carbon dioxide is held in the form of a carbon dioxide aqueous solution, that is, carbonated water in the carbon dioxide slow-release pack for skin is preferable since the handling of the carbon dioxide slow-release pack for skin is simplified.

In addition, the inverse diffusion filling method is also preferable since there is no need to provide an inlet or a port portion for a carbon dioxide gas or to hermetically seal the carbon dioxide gas, and thus the pack structure can be simplified.

The carbon dioxide concentration in the carbon dioxide aqueous solution which is introduced into the carbon dioxide slow-release pack for skin according to the present embodiment is preferably 300 to 4,000 ppm, and more preferably 1,000 to 4,000 ppm.

In addition, as the amount of carbon dioxide filled in the carbon dioxide slow-release pack for skin according to the present embodiment, for example, a capacity of the carbon dioxide slow-release pack for skin swelling appropriately is exemplified in a case where the carbon dioxide is only in a gaseous state.

In addition, in a case where carbon dioxide is introduced in a gaseous state into the carbon dioxide slow-release pack for skin having a sealed system by the above-described inverse diffusion method, for example, a capacity of the carbon dioxide slow-release pack for skin which appropriately swells to include the air immediately before being placed under a high-concentration carbon dioxide atmosphere and further swells due to the diffusion of the carbon dioxide after being placed under the high-concentration carbon dioxide atmosphere is exemplified as the amount of carbon dioxide.

In order to increase a carbon dioxide slow-release time or a carbon dioxide slow-release amount, a substance having adsorbability and slow-releasability with respect to carbon dioxide may be stored in the carbon dioxide slow-release pack for skin. Examples of the substance having adsorbability and slow-releasability with respect to carbon dioxide include silica gel, alumina, zeolite, and activated carbon in a case where the substance is a porous body. In a case where the substance having adsorbability and slow-releasability with respect to carbon dioxide is a resin, those having a structure with a high carbonyl group density as a chemical structure, such as a polylactic acid (PLA) and a polymethyl methacrylic acid (PMMA), can be preferably used since those have high adsorbability and slow-releasability with respect to carbon dioxide. In this case, those are preferably used in a form having a large specific surface area such as a granular form, a film form, or a fibrous form.

In addition, in a case where carbon dioxide in a state of carbonated water is filled in the carbon dioxide slow-release pack for skin, the amount of the carbonated water to be enclosed in the carbon dioxide slow-release pack for skin may be 100%, preferably 30% to 90%, and more preferably 50% to 80% in terms of volume ratio with respect to the volume when the carbon dioxide slow-release pack for skin is filled with pure water. In a case where the volume ratio is equal to or less than the upper limit value, it is possible to suppress overflow of the carbonated water from the introduction port, thereby solving difficulties in handling in a case where the carbonated water is introduced into the carbon dioxide slow-release pack for skin and is then sealed. In a case where the volume ratio is equal to or more than the lower limit value, the amount of carbon dioxide to be slowly released from the carbon dioxide slow-release pack for skin can be increased, and thus the blood circulation promoting effect for skin by the percutaneous absorption of the carbon dioxide can be further improved.

The carbon dioxide slow-release pack for skin according to the present embodiment is a bag-shaped container in which at least the resin sheet (A) disposed on the skin side has a carbon dioxide permeation property, and can be easily used by sealing carbon dioxide therein and being brought into close contact with the skin with water interposed between the carbon dioxide slow-release pack for skin and the skin.

Hereinafter, respective constituent members of the carbon dioxide slow-release pack for skin according to the present embodiment will be described.

<Resin Sheet (A)>

In the resin sheet (A) according to the present embodiment, the carbon dioxide permeability measured under the conditions of 23°C and a relative humidity of 0% according to JIS K7126-1 is 20 L/(m$^2$·day·atm) or more, and from the viewpoint of a further improvement in blood circulation promoting property and in sustainability of the blood circulation promoting effect, the carbon dioxide permeability is preferably 30 L/(m$^2$·day·atm) or more, more preferably 50 L/(m$^2$·day·atm) or more, even more preferably 100 L/(m$^2$·day·atm) or more, still more preferably 110 L/(m$^2$·day·atm) or

more, and yet still more preferably 120 L/ (m²·day·atm) or more. The upper limit value of the carbon dioxide permeability is not particularly limited, and is, for example, 1,000,000 L/(m²·day·atm) or less.

In the resin sheet (A) according to the present embodiment, the Oken-type air permeability measured at 23°C according to JIS P8117: 2009 is less than 5 μm/(Pa·s), and from the viewpoint of a further improvement in sustainability of the blood circulation promoting effect, the Oken-type air permeability is preferably less than 1 μm/(Pa·s), more preferably less than 0.5 μm/(Pa·s), and even more preferably less than 0.05 μm/(Pa·s).

The resin sheet (A) according to the present embodiment preferably further satisfies the following requirement (A-3) from the viewpoint of improving the shape conformability of the carbon dioxide slow-release pack for skin to the skin.

(A-3) Young's modulus E at 23°C is 10 MPa to 500 MPa, preferably 50 MPa to 400 MPa, and more preferably 100 MPa to 300 MPa.

The resin sheet (A) according to the present embodiment preferably satisfies the requirement (A-3) since the resin sheet easily follows a wide area of the human body surface or a three-dimensional shape of the human body surface.

In a case where the conformability to a curved surface of the human body is good, the generation of a gap between the resin sheet (A) and the skin can be suppressed, or the contact area can be increased. Accordingly, a close contact state to the wide human body surface can be stably secured, and transdermal absorption of carbon dioxide can be improved.

In addition, in a case where the resin sheet (A) according to the present embodiment satisfies the requirement (A-3), it is possible to suppress unstable supply of carbon dioxide to the skin due to breaking of the bag or rapid leakage of the carbon dioxide from the bag even in a case where high-concentration carbonated water is generated in the bag or in a case where a carbon dioxide gas having a pressure higher than the atmospheric pressure is introduced, and thus the internal pressure of the bag increases at least temporarily.

The resin sheet (A) according to the present embodiment preferably further satisfies the following requirement (A-4) from the viewpoint of improving the shape conformability of the carbon dioxide slow-release pack for skin to the skin.

(A-4) tensile elongation at break at 23°C is 30% or more, preferably 50% or more, more preferably 100% or more, even more preferably 150% or more, and particularly preferably 200% or more.

The resin sheet (A) according to the present embodiment preferably satisfies the requirement (A-4) since the resin sheet (A) easily follows a three-dimensional shape of the human body surface.

The upper limit value of the tensile elongation at break of the resin sheet (A) according to the present embodiment at 23°C is not particularly limited, and is, for example, 2,000% or less.

The resin sheet (A) according to the present embodiment preferably satisfies both the requirements (A-3) and (A-4). Accordingly, the flexibility and the shape conformability of the resin sheet (A) are further improved, and the adhesion to the human body surface, the feeling of fitting, and the feeling of wearing can be further improved.

A thickness t of the resin sheet (A) may be selected in consideration of a gas permeability coefficient, flexibility, and shape conformability, and is preferably 0.01 to 5 mm, and more preferably 0.03 to 2 mm.

In a case where the thickness t is equal to or more than the lower limit value, the film strength is improved, and the occurrence of bag breaking can be further suppressed when the bag is formed. In a case where the thickness t is equal to or less than the upper limit value, the carbon dioxide permeation property can be improved, or softness is improved or the shape conformability to the human body surface can be further improved when the bag is formed and brought into close contact with the human body surface.

The resin sheet (A) preferably has hydrophilicity on the skin contact side in order to allow moisture to be easily interposed between the carbon dioxide slow-release pack for skin and the skin. In a case where a hydrophobic polyolefin-based material is used, a film or sheet thereof can be subjected to a corona treatment, an atmospheric pressure plasma treatment, or the like for a hydrophilization treatment.

Furthermore, fine irregularities may be formed on the surface of the resin sheet (A) by embossing or the like so as to suppress blocking between the resin sheets or to impart a slipping property.

As the resin sheet (A) used in the carbon dioxide slow-release pack for skin according to the present embodiment, for example, a non-porous film or sheet or a microporous film or sheet having micropores can be used.

(Non-Porous Film or Sheet)

The non-porous film or sheet means a resin film or sheet in which the raw material resin itself has a high carbon dioxide permeation property without fine holes, or by reducing the thickness, the carbon dioxide permeation property or flexibility can be increased while maintaining the strength. Examples of the resin having such characteristics include polyolefin resins such as polyethylene, polypropylene, ethylene-based copolymers, propylene-based copolymers, butene-based copolymers, and 4-methyl-1-pentene-based polymers, styrene-based elastomers such as SEBS, SEPS, and SIS, fluorine-based resins such as PTFE, FEP, ETFE, and PFA, and silicone-based resins such as polydimethylsiloxane (PDMS). Polybutadiene is also preferable due to its high flexibility and carbon dioxide permeation property. Polyurethane and polyether block amide copolymers can be preferably used since these have flexibility and can be easily formed into a thin film.

A film or sheet using these resins may be a single layer or a multilayer with other resins.

Among these, polyethylene, ethylene-based copolymers, propylene-based copolymers, 4-methyl-1-pentene-based polymers, styrene-based elastomers, silicone, polyurethane, and polyether block amide copolymers can be preferably used as a resin having a high carbon dioxide permeation property, an appropriate Young's modulus, large tensile elongation at break, and excellent shape conformability. Particularly, polyethylene, ethylene-based copolymers, propylene copolymers, styrene-based elastomers, and polyether block amide copolymers having excellent heat sealability are preferable, and the reason for this is that since a high sealing strength can be maintained in the formation of the carbon dioxide slow-release pack for skin by heat sealing, a sealed portion is less likely to break even in a case where high-concentration carbonated water is introduced into the carbon dioxide slow-release pack for skin and sealed, and then the internal pressure of the carbon dioxide slow-release pack for skin is increased, whereby the reliability of the carbon dioxide slow-release pack for skin is maintained. Among the 4-methyl-1-pentene-based polymers, copolymers including a constituent unit derived from 4-methyl-1-pentene and a constituent unit derived from an $\alpha$-olefin having 2 to 20 carbon atoms other than 4-methyl-1-pentene are preferable due to excellent flexibility, shape conformability, heat sealing strength, and the like thereof, and 4-methyl-1-pentene/propylene copolymers are more preferable.

The resin sheet (A) is preferably a film or sheet formed of polyolefin, that is, a polyolefin film or polyolefin sheet, more preferably a polyolefin film or polyolefin sheet including at least one selected from polyethylene, an ethylene-based copolymer, a propylene-based copolymer, and a 4-methyl-1-pentene-based polymer, even more preferably a polyolefin film or polyolefin sheet including a copolymer including a constituent unit derived from 4-methyl-1-pentene and a constituent unit derived from an $\alpha$-olefin having 2 to 20 carbon atoms other than 4-methyl-1-pentene, and still more preferably a polyolefin film or polyolefin sheet including a 4-methyl-1-pentene/propylene copolymer.

(Microporous Film or Sheet)

The microporous film or sheet used as the resin sheet (A) according to the present embodiment is preferably a film which has many fine holes allowing the front and back sides to communicate with each other, is capable of preferentially transmitting carbon dioxide in a gaseous or carbonated water state through the fine holes, and has flexibility and good shape conformability. Examples of the microporous film or sheet include polyolefin-based microporous films, filler-containing polyolefin-based microporous films, and foamed sheets having communication holes. Particularly, foamed sheets formed of a 4-methyl-1-pentene-based polymer having excellent flexibility and shape conformability are preferable, foamed sheets formed of a copolymer including a constituent unit derived from 4-methyl-1-pentene and a constituent unit derived from an $\alpha$-olefin having 2 to 20 carbon atoms other than 4-methyl-1-pentene are more preferable, and foamed sheets formed of a 4-methyl-1-pentene/propylene copolymer are even more preferable. As the foamed sheet, for example, a foamed extrusion-molded sheet is preferable.

As the microporous film or sheet used as the resin sheet (A) according to the present embodiment, one or more selected from a foamed sheet formed of a 4-methyl-1-pentene-based polymer, a polypropylene-based microporous film, and a filler-containing polyolefin-based microporous film are preferably included.

The polypropylene-based microporous film or the filler-containing polyolefin-based microporous film is not particularly limited, and examples thereof include product name: MICROPOROUS FILM manufactured by 3M Japan Limited, a sheet having fine holes formed by biaxially stretching a polypropylene sheet filled with fine calcium carbonate (manufactured by Tokuyama Corporation, product name: NF SHEET), a polyolefin-based microporous film (moisture-permeable sheet manufactured by Mitsubishi Chemical Corporation, product name: KTF and trade name EXEPOL), and a microporous film formed of polyethylene and an inorganic filler (manufactured by Mitsui Hygiene Materials (Thailand): trade name ESPOIR).

Examples of the non-polyolefin-based film include PTFE-based microporous film or sheets.

(4-Methyl-1-Pentene-Based Polymer)

The resin sheet (A) according to the present embodiment preferably contains a 4-methyl-1-pentene-based polymer.

Examples of the 4-methyl-1-pentene-based polymer according to the present embodiment include a 4-methyl-1-pentene/$\alpha$-olefin copolymer (A3) including a constituent unit (A1) derived from 4-methyl-1-pentene and a constituent unit (A2) derived from an $\alpha$-olefin having 2 to 20 carbon atoms other than 4-methyl-1-pentene due to excellent flexibility and shape conformability.

Here, in the present embodiment, "$\alpha$-olefin having 2 to 20 carbon atoms" means that 4-methyl-1-pentene is not contained unless otherwise specified.

In the 4-methyl-1-pentene/$\alpha$-olefin copolymer (A3) according to the present embodiment, from the viewpoint of further improving the flexibility of the resin sheet (A), it is preferable that the content of the constituent unit (A1) is 10 mol% to 95 mol%, and the content of the constituent unit (A2) is 5 mol% to 90 mol% on the assumption that a total content of the constituent unit (A1) and the constituent unit (A2) is 100 mol%.

In addition, in the 4-methyl-1-pentene/$\alpha$-olefin copolymer (A3) according to the present embodiment, from the viewpoint of further improving the flexibility or mechanical characteristics of the resin sheet (A), it is more preferable that the content of the constituent unit (A1) is 10 mol% to 90 mol%, and the content of the constituent unit (A2) is 10 mol% to 90 mol%, it is even more preferable that the content of the constituent unit (A1) is 15 mol% to 90 mol%, and the content of the constituent unit (A2) is 10 mol% to 85 mol%, it is still more preferable that the content of the constituent unit (A1) is 40 mol% to 90 mol%, and the content of the constituent unit (A2) is 10 mol% to 60 mol%, it is yet more preferable that the content of the constituent unit (A1) is 50 mol% to 90 mol%, and the content of the constituent unit (A2) is 10 mol% to 50 mol%, and it is particularly preferable that the content of the constituent unit (A1) is 60 mol% to 90 mol%, and the content of the constituent unit (A2) is 10 mol% to 40 mol% on the assumption that a total content of the constituent unit (A1) and the constituent unit (A2) is 100 mol%.

In the present embodiment, examples of the $\alpha$-olefin having 2 to 20 carbon atoms used in the 4-methyl-1-pentene/$\alpha$-olefin copolymer (A3) include a linear or branched $\alpha$-olefin, a cyclic olefin, an aromatic vinyl compound, a conjugated diene, and a functionalized vinyl compound, and a linear $\alpha$-olefin is preferable.

The number of carbon atoms of the linear $\alpha$-olefin is preferably 2 to 10, and more preferably 2 or 3. Examples of the linear $\alpha$-olefin include ethylene, propylene, 1-butene, 1-pentene, 1-hexene, 1-octene, 1-decene, 1-undecene, 1-dodecene, 1-tetradecene, 1-hexadecene, 1-octadecene, and 1-eicosene. One or more selected from ethylene, propylene, 1-butene, 1-pentene, 1-hexene, 1-octene, and 1-decene are preferable, at least one selected from ethylene and propylene is more preferable, and propylene is even more preferable.

The number of carbon atoms of the branched $\alpha$-olefin is preferably 5 to 20, and more preferably 5 to 15. Examples of the branched $\alpha$-olefin include 3-methyl-1-butene, 3-methyl-1-pentene, 3-ethyl-1-pentene, 4,4-dimethyl-1-pentene, 4-methyl-1-hexene, 4,4-dimethyl-1-hexene, 4-ethyl-1-hexene, and 3-ethyl-1-hexene.

The number of carbon atoms of the cyclic olefin is preferably 5 to 15. Examples of the cyclic olefin include cyclopentene, cyclohexene, cycloheptene, norbornene, 5-methyl-2-norbornene, tetracyclododecene, and vinylcyclohexane.

Examples of the aromatic vinyl compound include mono- or polyalkylstyrenes such as styrene, $\alpha$-methylstyrene, o-methylstyrene, m-methylstyrene, p-methylstyrene, o,p-dimethylstyrene, o-ethylstyrene, m-ethylstyrene, and p-ethylstyrene.

The number of carbon atoms of the conjugated diene is preferably 4 to 20, and more preferably 4 to 10. Examples of the conjugated diene include 1,3-butadiene, isoprene, chloroprene, 1,3-pentadiene, 2,3-dimethylbutadiene, 4-methyl-1,3-pentadiene, 1,3-hexadiene, and 1,3-octadiene.

Examples of the functionalized vinyl compound include hydroxyl group-containing olefins, halogenated olefins, unsaturated carboxylic acids such as (meth)acrylic acid, propionic acid, 3-butenoic acid, 4-pentenoic acid, 5-hexenoic acid, 6-heptenoic acid, 7-octenoic acid, 8-nonenoic acid, 9-decenoic acid, and 10-undecenoic acid, and their acid anhydrides or acid halides, unsaturated amines such as allylamine, 5-hexenamine, and 6-heptenamine, (2,7-octadienyl) succinic anhydride, pentapropenyl succinic anhydride, unsaturated epoxy compounds, and ethylenically unsaturated silane compounds.

Examples of the hydroxyl group-containing olefins include linear or branched terminal hydroxylated $\alpha$-olefins having 2 to 20, preferably 2 to 15, carbon atoms.

Examples of the halogenated olefins include linear or branched halogenated $\alpha$-olefins having 2 to 20, preferably 2 to 15, carbon atoms.

These $\alpha$-olefins having 2 to 20 carbon atoms can be used alone or in combination of two or more types thereof. Among these, ethylene and propylene are suitable, and propylene is particularly preferably used since the flexibility or the like can be further improved.

The 4-methyl-1-pentene/$\alpha$-olefin copolymer (A3) may include other constituent units in addition to the constituent units (A1) and (A2) within a range not impairing the object of the present invention. Examples of other constitutions include constituent units derived from non-conjugated polyenes.

Examples of the non-conjugated polyenes include linear, branched, or cyclic dienes having preferably 5 to 20, and more preferably 5 to 10 carbon atoms, various norbornenes, and norbornadienes. Among these, 5-vinylidene-2-norbornene and 5-ethylidene-2-norbornene are preferable.

The intrinsic viscosity [$\eta$] of the 4-methyl-1-pentene-based polymer according to the present embodiment in decalin at 135°C is preferably 0.01 to 5.0 dL/g, more preferably 0.1 to 4.0 dL/g, even more preferably 0.5 to 3.0 dL/g, and particularly preferably 1.0 to 2.8 dL/g from the viewpoint of further improving the flexibility and mechanical strength of the resin sheet (A).

The density of the 4-methyl-1-pentene-based polymer according to the present embodiment, measured according to ASTM D 1505 (substitution method in water), is preferably 0.810 to 0.850 g/cm$^3$, more preferably 0.820 to 0.850 g/cm$^3$, and even more preferably 0.830 to 0.850 g/cm$^3$.

The 4-methyl-1-pentene-based polymer according to the present embodiment can be produced by various methods. For example, the 4-methyl-1-pentene-based polymer can be manufactured using known catalysts such as magnesium-supported titanium catalysts; metallocene catalysts described in International Publication No. WO01/53369, International Publication No. WO01/027124, Japanese Published Patent Application A-H03-193796, and Japanese Published Patent Application A-H02-41303; and metallocene compound-containing olefin polymerization catalysts described in International Publication No. WO2011/055803.

The content of the 4-methyl-1-pentene-based polymer in the resin sheet (A) according to the present embodiment is not particularly limited. On the assumption that the whole resin sheet (A) is 100 mass%, the content is preferably 50 mass% or more, more preferably 60 mass% or more, even more preferably 65 mass% or more, and particularly preferably 70 mass% or more, and is preferably 100 mass% or less, more preferably 99.5 mass% or less, even more preferably 99 mass% or less, still more preferably 98 mass% or less, and particularly preferably 97 mass% or less.

This makes it possible to obtain a resin sheet (A) which is further improved in balance among flexibility, shape retainability, shape conformability, light weight property, mechanical characteristics, handleability, appearance, moldability, moisture resistance, and the like.

In a case where the resin sheet (A) contains a 4-methyl-1-pentene-based polymer in the carbon dioxide slow-release pack for skin according to the present embodiment, the resin sheet (A) according to the present embodiment preferably includes a layer formed of a 4-methyl-1-pentene-based polymer and a layer formed of a thermoplastic elastomer from the viewpoint of suppressing permanent deformation of the pack, improving bending resistance of the pack under a low temperature environment (for example, lower than 20°C), and further improving durability of the pack.

Such a multilayer sheet can be produced by, for example, a co-extrusion molding method, and specifically, it has the following configuration.

(1) (skin side) layer formed of 4-methyl-1-pentene-based polymer/layer formed of thermoplastic elastomer/layer formed of 4-methyl-1-pentene-based polymer = 25/50/25 $\mu$m, total thickness 100 $\mu$m

(2) (skin side) layer formed of 4-methyl-1-pentene-based polymer/layer 1 formed of thermoplastic elastomer/layer 2 formed of thermoplastic elastomer/layer 3 formed of thermoplastic elastomer/layer formed of 4-methyl-1-pentene-based polymer = 25/10/30/10/25 $\mu$m, total thickness 100 $\mu$m

It has been confirmed that in a case where a carbon dioxide slow-release pack for skin is formed using a resin sheet (A) having the configuration (1) or (2) with the use of a styrene-based elastomer as the thermoplastic elastomer, the pack has good conformability and a good blood circulation promoting property in any case, and the occurrence of pinholes in a bent portion is suppressed when the pack is bent at 5°C under a low temperature environment.

In addition, the durability of the carbon dioxide slow-release pack for skin can be further increased by forming a multilayer with a fibrous reinforcing material to be described later.

Here, examples of the thermoplastic elastomer include olefin-based elastomers, styrene-based elastomers, urethane-based elastomers, ester-based elastomers, and amide-based elastomers.

These thermoplastic elastomers may be used alone or in combination of two or more types thereof. Among these thermoplastic elastomers, particularly, styrene-based elastomers have a high carbon dioxide permeability and appropriate compatibility with the 4-methyl-1-pentene-based polymer, and easily secure the interlayer adhesive force in the formation of a co-extruded multilayer sheet formed of the layers . In addition, in a case where a bag-shaped carbon dioxide slow-release pack for skin is formed by heat sealing, the pack can be preferably used since the peeling strength of a heat sealed portion can be kept high, and thus the bag is hardly broken, and the reliability of the carbon dioxide slow-release pack can be increased. Among styrene-based elastomers, hydrogenated styrene-based elastomers having a low styrene content can be preferably used since these have good compatibility with the 4-methyl-1-pentene-based polymer and a high carbon dioxide permeability. Specific examples of such hydrogenated styrene-based elastomer products include brand KRATON G1657MS; manufactured by Kraton Polymers Japan Ltd., brand TUFTEC H1221, brand TUFTEC H1062, brand TUFTEC H1521, brand TUFTEC 1052, and brand S. O.E. 1606; manufactured by Asahi Kasei Corporation, and brand SEPTON 2004F and brand HYBRAR 7311F; manufactured by KURARAY CO., LTD.

The carbon dioxide slow-release pack for skin according to the present embodiment may further include a fibrous reinforcing material on the surface of the resin sheet (A) or inside the resin sheet (A) from the viewpoint of further improving the durability.

The fibers of the fibrous reinforcing material are not particularly limited, and examples thereof include: natural fibers such as cotton, silk, and wool; polyamide fibers such as polyamide 6 and polyamide 66; polyester fibers such as polyethylene terephthalate, polytrimethylene terephthalate, and polytetramethylene terephthalate; and polyurethane fibers.

Examples of the warp knitted fabric of the fibrous reinforcing material include a power net structure, a satin net, a Raschel lace, and a two-way tricot, and a power net structure having an appropriate stretching property is preferable.

Further, nonwoven fabric may be used as the fibrous reinforcing material. As the nonwoven fabric, stretchable nonwoven fabric made of polypropylene fibers and urethane fibers may be used.

The thickness of the fibrous reinforcing material is not particularly limited, and is for example, 0.01 mm to 0.5 mm. In a case where the fibrous reinforcing material has a power net structure, the mesh size thereof is not particularly limited. For example, the mesh size is 0.5 mm to 10 mm.

In the present embodiment, by heat laminating the fibrous reinforcing material on the surface of the resin sheet (A), the fibrous reinforcing material can be provided on the surface of the resin sheet (A). In addition, by placing the fibrous reinforcing material between two resin sheets (A) and heat laminating the resulting laminate, the fibrous reinforcing material can be provided inside the resin sheet (A).

<Resin Sheet (B)>

The water resistance of the resin sheet (B) according to the present embodiment, measured at 23°C according to JIS L1092A: 2009, is 350 mbar or more, and preferably 400 mbar or more. The water resistance of the resin sheet (B) according to the present embodiment is preferably equal to or more than the lower limit value since the leakage of carbon dioxide from the inside of the carbon dioxide slow-release pack for skin is further suppressed, and loss of the carbon dioxide enclosed in the bag is reduced.

The upper limit value of the water resistance is, for example, 10 bar or less, and is not particularly limited.

In the resin sheet (B) according to the present embodiment, the Oken-type air permeability measured at 23°C according to JIS P8117: 2009 is less than 5 $\mu$m/ (Pa·s), preferably less than 1 $\mu$m/ (Pa·s), more preferably less than 0.5 $\mu$m/(Pa·s), and even more preferably less than 0.05 $\mu$m/(Pa·s).

The Oken-type air permeability of the resin sheet (B) according to the present embodiment is preferably less than the upper limit value since the leakage of carbon dioxide from the inside of the carbon dioxide slow-release pack for skin is suppressed, and loss of the carbon dioxide enclosed in the bag is reduced.

The resin sheet (B) according to the present embodiment preferably further satisfies the following requirement (B-3) from the viewpoint of improving the shape conformability of the carbon dioxide slow-release pack for skin to the skin.

(B-3) Young's modulus E at 23°C is 10 MPa to 500 MPa, preferably 50 MPa to 400 MPa, and more preferably 100 MPa to 300 MPa.

The resin sheet (B) according to the present embodiment preferably satisfies the requirement (B-3) since the resin sheet easily follows a wide area of the human body surface or a three-dimensional shape of the human body surface.

In a case where the conformability to a curved surface of the human body is good, the generation of a gap between the resin sheet (A) and the skin can be suppressed, or the contact area can be increased. Accordingly, a close contact state to the wide human body surface can be stably secured, and transdermal absorption of carbon dioxide can be improved.

In addition, in a case where the resin sheet (B) according to the present embodiment satisfies the requirement (B-3), it is possible to suppress unstable supply of carbon dioxide to the skin due to breaking of the bag or rapid leakage of the carbon dioxide from the bag even in a case where high-concentration carbonated water is generated in the bag or in a case where a carbon dioxide gas having a pressure higher than the atmospheric pressure is introduced, and thus the internal pressure of the bag increases at least temporarily.

The resin sheet (B) according to the present embodiment preferably further satisfies the following requirement (B-4) from the viewpoint of improving the shape conformability of the carbon dioxide slow-release pack for skin to the skin.

(B-4) tensile elongation at break at 23°C is 30% or more, preferably 100% or more, more preferably 150% or more, and even more preferably 200% or more.

The resin sheet (B) according to the present embodiment preferably satisfies the requirement (B-4) since the resin sheet (B) easily follows a three-dimensional shape of the human body surface.

The upper limit value of the tensile elongation at break of the resin sheet (B) according to the present embodiment at 23°C is not particularly limited, and is, for example, 2,000% or less.

The resin sheet (B) according to the present embodiment preferably satisfies both the requirements (B-3) and (B-4). Accordingly, the resin sheet (B) has appropriate flexibility and shape conformability, and the adhesion to the human body surface, the feeling of fitting, and the feeling of wearing can be further improved.

As the resin sheet (B) used in the carbon dioxide slow-release pack for skin according to the present embodiment, for example, a resin film or sheet having a low carbon dioxide permeation property and hardly transmitting carbonated water is preferable. A resin film or sheet having flexibility is also preferable.

Examples of means for reducing the carbon dioxide permeation property of the resin sheet (B) include: a method of increasing the thickness of the resin sheet (B) itself; a method of providing a resin layer having a high carbon dioxide barrier property on one or both surfaces of the resin sheet (B); and a method of providing a carbon dioxide barrier thin film layer such as an inorganic film of aluminum or oxide-based ceramics on one or both surfaces of the resin sheet (B).

In a case where the resin sheets (A) and (B) are heat sealed in the formation of the carbon dioxide slow-release pack for skin according to the present embodiment, the resin sheet (B) is preferably a material having heat sealability to the resin sheet (A).

For example, in a case where a resin for forming the resin sheet (A) is polyethylene, a resin having good compatibility with the polyethylene, such as polyethylene, an ethylene-based copolymer, SEBS, or SEPS, is preferably selected as a resin for forming the resin sheet (B). In a case where a resin for forming the resin sheet (A) is a propylene-based copolymer, a resin having good compatibility with the propylene-based copolymer, such as polypropylene, a propylene-based copolymer, SEBS, or SEPS, is preferably selected as a resin for forming the resin sheet (B).

In a case where a resin for forming the resin sheet (A) is SEBS or SEPS, a resin having good compatibility with the SEBS or SEPS, such as polyethylene, an ethylene-based copolymer, polypropylene, a propylene-based copolymer, or a styrene-based elastomer, is preferably selected as a resin for forming the resin sheet (B).

In a case where a resin for forming the resin sheet (A) is a 4-methyl-1-pentene/$\alpha$-olefin copolymer (A3), for example, a 4-methyl-1-pentene/$\alpha$-olefin copolymer (A3), polypropylene, or a polypropylene-based copolymer is preferably used as a resin for forming the resin sheet (B) since the heat sealing strength is easily secured.

The embodiments of the present invention have been described as above. These are merely examples of the present invention, and various configurations other than the above-described configurations can be employed.

Hereinafter, the present invention will be described in detail based on examples, but is not limited to these examples.

1. Measuring Method

(1) Carbon Dioxide Permeability of Resin Sheet

The carbon dioxide permeability of a resin sheet was measured under the conditions of 23°C and a relative humidity of 0% according to JIS K7126-1. As a device measuring the carbon dioxide permeability, a carbon dioxide permeability measuring device (manufactured by Toyo Seiki Seisaku-sho, Ltd., product name: differential pressure method gas permeability measuring device) was used. The measurement was performed twice, and an average of the measurement results was employed.

Here, in Tables 1 and 2, those having a carbon dioxide permeability in a range of 1,000 L/(m$^2$·day·atm) to 1,000,000 L/(m$^2$·day·atm) are expressed as "1,000 or more".

(2) Oken-Type Air Permeability of Resin Sheet

The Oken-type air permeability of a resin sheet was measured under an atmospheric environment at 23°C according to JIS P8117: 2009. As a device measuring the Oken-type air permeability, an Oken-type air permeability tester (manufactured by KUMAGAI RIKI KOGYO Co., Ltd., product name: Oken-type smoothness/air permeability tester (water column type)) was used. The measurement was performed ten times, and an average of the measurement results was employed.

(3) Water Resistance of Resin Sheet

The water resistance of a resin sheet was measured under an environment of 23°C and a relative humidity of 50% according to JIS L1092A: 2009. As a device measuring the water resistance, a water resistance tester (manufactured by TEXTEST AG, product name: Automatic Hydrostatic Head Tester FX3000 Hydrotester IV) was used. The pressing speed was 60 mbar/min. The measurement was performed five times, and an average of the measurement results was employed.

Since the non-porous film having a thickness of 0.01 mm or less and the porous film having a thickness of 0.04 mm or less shown in Table 1 had a weak strength, and thus were measured by supporting with a resin mesh.

Here, in Tables 1 and 2, those having water resistance in a range of 400 mbar or more were expressed as "400 or more".

(4) Tensile Test of Resin Sheet

The tensile properties of a resin sheet (Young's modulus E, tensile elongation at break, and tensile strength at break) were respectively measured at 3 points in MD and TD directions with the conditions of shape of test piece: strip shape, width of test piece: 15 mm, distance between chucks: 30 mm, and tensile speed: 1,000%/min under an environment of 23°C and a relative humidity of 50%, and an average of the measurement results was employed.

2. Evaluation Method

(1) Evaluation of Conformability of Carbon Dioxide Slow-Release Pack

The shape conformability of the obtained carbon dioxide slow-release pack was evaluated with the following criteria.

A: The surface of the carbon dioxide slow-release pack on the side of the resin sheet (A) was soft and had a good touch, the adhesion was good when the carbon dioxide slow-release pack was brought into contact with the skin, and the conformability (particularly, conformability to fine irregularities on the skin surface) to the shape of the skin was good. Furthermore, even in a case where the pack was brought into contact with the skin for a long period of time, elastic recovery of the shape was suppressed, and the shape retainability was good.

B: The surface of the carbon dioxide slow-release pack on the side of the resin sheet (A) was soft and had a good touch, the adhesion was good when the carbon dioxide slow-release pack was brought into contact with the skin, and the conformability to the shape of the skin was good.

D: The surface of the carbon dioxide slow-release pack on the side of the resin sheet (A) was hard and had a poor touch, the adhesion was poor when the carbon dioxide slow-release pack was brought into contact with the skin, and the conformability to the shape of the skin was poor.

(2) Evaluation of Blood Circulation Promoting Property of Carbon Dioxide Slow-Release Pack

The blood circulation promoting property of the carbon dioxide slow-release pack was evaluated by the following method.

A predetermined amount of water was sprayed on the skin on the inside of a forearm portion with a spray before the obtained carbon dioxide slow-release pack was disposed on the skin on the inside of the forearm portion. Immediately before and immediately after the spraying, the moisture content of the skin was measured using product name: BELULU skin checker (product number: KRD1042), manufactured by Beautiful Angel Inc. (Kireido). Immediately after spraying the water on the skin with a spray, the carbon dioxide slow-release pack was disposed on the skin on the inside of the forearm portion. Next, the flushing degree of the skin was visually observed at 5 minutes, 10 minutes, and 20 minutes after the carbon dioxide slow-release pack was disposed on the skin on the inside of the forearm portion, and the blood circulation promoting property was evaluated with the following criteria.

A: The skin is distinctly flushed, and the boundary of adhesion is clearly seen.
B: The skin is flushed, and the boundary of adhesion is clearly seen.
C: The skin is flushed, but the boundary is not clear.
D: No flushing is observed on the skin.

3. Used Resin Sheet

Tables 1 and 2 respectively show the resin sheets (A) and (B) used in the examples and comparative examples.

[Table 1]

| | | Manufacturer/ Product Name | Presence or Absence of Porosity | Thickness | Carbon Dioxide Permeability (23°C×relative humidity 0%) | Oken-Type Air Permeability (23°C) | Water Resistance (23°C) | Tensile Properties | | |
|---|---|---|---|---|---|---|---|---|---|---|
| | | | | | | | | Young's Modulus | Tensile Elongation at Break | Tensile Strength at Break |
| | | | | [mm] | [L/($m^2$ day·atm)] | [μm/(Pa·s)] | [mbar] | [MPa] | [%] | [MPa] |
| PE-1 | Low-Density Polyethylene Film | - | Non-Porous | 0.01 | 110 | less than 0.05 | 400 or more | 110 | 150 | 16 |
| PE-2 | Low-Density Polyethylene Film | - | Non-Porous | 0.04 | 20 | less than 0.05 | 400 or more | 180 | 440 | 16 |
| PE-3 | Low-Density Polyethylene Film | - | Non-Porous | 0.08 | 10 | less than 0.05 | 400 or more | 190 | 460 | 16 |
| PE-4 | Polyethylene-Inorganic Filler-Based Microporous Film | Mitsui Hygiene Materials (Thailand)/ ESPOIR | Porous | 0.019 | 1,000 or more | 0.2 | 370 | 80 | 240 | 4 |
| PE-5 | High-Density Polyethylene Nonwoven Fabric | DuPont/ TYVEK, 1056-D | Porous | 0.06 | 1,000 or more | 3.8 | 56 | 350 | 20 | 39 |
| PP-1 | Propylene-Based Copolymer Film | TOYOBO/PYLEN FILM, P1128 | Non-Porous | 0.06 | 5 | less than 0.05 | 400 or more | 560 | 470 | 19 |
| PP-2 | Polypropylene-Based Microporous Film | 3M Japan/ MICROPOROUS FILM | Porous | 0.04 | 1,000 or more | 1.2 | 400 or more | 38 | 180 | 15 |
| PMP-1 | Film Including 4-Methyl-1-Pentene Copolymer and Polybutene-1 | RIKEN FABRO/ F.O.R. WRAP 45 | Non-Porous | 0.01 | 650 | less than 0.05 | 400 or more | 90 | 140 | 27 |
| PMP-2 | Film of 4-Methyl-1-Pentene/Propylene Copolymer 1 | - | Non-Porous | 0.05 | 110 | less than 0.05 | 400 or more | 130 | 260 | 16 |

EP 3 698 800 A1

| | | Manufacturer/ Product Name | Presence or Absence of Porosity | Thickness | Carbon Dioxide Permeability (23°C×relative humidity 0%) | Oken-Type Air Permeability (23°C) | Water Resistance (23°C) | Tensile Properties | | |
|---|---|---|---|---|---|---|---|---|---|---|
| | | | | | | | | Young's Modulus | Tensile Elongation at Break | Tensile Strength at Break |
| | | | | [mm] | [L/(m² day·atm)] | [μm/(Pa·s)] | [mbar] | [MPa] | [%] | [MPa] |
| PMP-3 | Film of 4-Methyl-1-Pentene/Propylene Copolymer 1 | - | Non-Porous | 0.1 | 55 | less than 0.05 | 400 or more | 110 | 400 | 25 |
| PMP-4 | Film of 4-Methyl-1-Pentene/Propylene Copolymer 2 | - | Non-Porous | 0.05 | 110 | less than 0.05 | 400 or more | 380 | 250 | 23 |
| PMP-5 | Film of 4-Methyl-1-Pentene/Propylene Copolymer 3 | - | Non-Porous | 0.1 | 55 | less than 0.05 | 400 or more | 250 | 320 | 24 |
| PMP-6 | Foamed Sheet of 4-Methyl-1-Pentene/Propylene Copolymer 1 | - | Porous | 1 | 1,000 or more | 0.35 | 23 | 22 | 300 | 3.1 |
| PMP-7 | Foamed Sheet of 4-Methyl-1-Pentene/Propylene Copolymer 1 | - | Porous | 0.5 | 1,000 or more | 85 | 8 | 48 | 210 | 3.5 |
| PMP-9 | Three-Layer Film Including 4-Methyl-1-Pentene/Propylene Copolymer Layer and Thermoplastic Elastomer (hydrogenated styrene-based elastomer) Layer | - | Non-Porous | 0.1 | 66 | less than 0.05 | 400 or more | 280 | 290 | 15 |

| | Manufacturer/ Product Name | Presence or Absence of Porosity | Thickness | Carbon Dioxide Permeability (23°C×relative humidity 0%) | Oken-Type Air Permeability (23°C) | Water Resistance (23°C) | Tensile Properties | | |
|---|---|---|---|---|---|---|---|---|---|
| | | | | | | | Young's Modulus | Tensile Elongation at Break | Tensile Strength at Break |
| | | | [mm] | [L/(m² day·atm)] | [μm/(Pa·s)] | [mbar] | [MPa] | [%] | [MPa] |
| PMP-10 | Multilayer Sheet Including 4-Methyl-1-Pentene/Propylene Copolymer Layer, Thermoplastic Elastomer (hydrogenated styrene-based elastomer) Layer, and Fibrous Reinforcina Material (power net structure) | - | Non-Porous | 0.26 | 33 | less than 0.05 | 400 or more | 130 | 140 | 9 |
| PMP-11 | Multilayer Sheet Including 4-Methyl-1-Pentene/Propylene Copolymer Layer, Thermoplastic Elastomer (hydrogenated styrene-based elastomer) Layer, and Fibrous Reinforcing Material (stretchable nonwoven fabric) | - | Non-Porous | 0.28 | 30 | less than 0.05 | 400 or more | 150 | 330 | 9 |

[Table 2]

| | | Manufacturer/ Product Name | Presence or Absence of Porosity | Thickness | Carbon Dioxide Permeability (23°C×relative humidity 0%) | Oken-Type Air Permeability (23°C) | Water Resistance (23°C) | Tensile Properties | | |
|---|---|---|---|---|---|---|---|---|---|---|
| | | | | | | | | Young's Modulus | Tensile Elongation at Break | Tensile Strength at Break |
| | | | | [mm] | [L/ (m²·day·atm)] | [μm/(Pa·s) ] | [mbar] | [MPa] | [%] | [MPa] |
| PE-2 | Low-Density Polyethylene Film | - | Non-Porous | 0.04 | 20 | less than 0.05 | 400 or more | 180 | 440 | 16 |
| PE-3 | Low-Density Polyethylene Film | - | Non-Porous | 0.08 | 10 | less than 0.05 | 400 or more | 190 | 460 | 16 |
| PMP-5 | Film of 4-Methyl-1-Pentene/Propylene Copolymer 3 | - | Non-Porous | 0.1 | 55 | less than 0.05 | 400 or more | 250 | 320 | 24 |
| PMP-8 | Sheet of 4-Methyl-1-Pentene/Propylene Copolymer 1 | - | Non-Porous | 0.6 | 6 | less than 0.05 | 400 or more | 83 | 520 | 17 |
| PMP-9 | Three-Layer Film Including 4-Methyl-1-Pentene/Propylene Copolymer Layer and ThermoplasticElastomer (hydrogenated styrene-based elastomer) Layer | - | Non-Porous | 0.1 | 66 | less than 0.05 | 400 or more | 280 | 290 | 15 |
| PMP-10 | Multilayer Sheet Including 4-Methyl-1-Pentene/Propylene Copolymer Layer, ThermoplasticElastomer (hydrogenated styrene-based elastomer) Layer, and Fibrous Reinforcing Material (power net structure) | - | Non-Porous | 0.26 | 33 | less than 0.05 | 400 or more | 130 | 140 | 9 |

(continued)

| | Manufacturer/ Product Name | Presence or Absence of Porosity | Thickness [mm] | Carbon Dioxide Permeability (23°C × relative humidity 0%) [L/(m²·day·atm)] | Oken-Type Air Permeability (23°C) [μm/(Pa·s)] | Water Resistance (23°C) [mbar] | Tensile Properties Young's Modulus [MPa] | Tensile Properties Tensile Elongation at Break [%] | Tensile Properties Tensile Strength at Break [MPa] |
|---|---|---|---|---|---|---|---|---|---|
| PMP-11 Multilayer Sheet Including 4-Methyl-1-Pentene/Propylene Copolymer Layer, ThermoplasticElastomer (hydrogenated styrene-based elastomer) Layer, and Fibrous Reinforcing Material (stretchable nonwoven fabric) | - | Non-Porous | 0.28 | 30 | less than 0.05 | 400 or more | 150 | 330 | 9 |
| PP-1 Propylene-Based Copolymer Film | TOYOBO/ PYLEN FILM, P1128 | Non-Porous | 0.06 | 5 | less than 0.05 | 400 or more | 560 | 470 | 19 |

[Examples 1 to 16, Comparative Examples 1 to 3, and Reference Examples 1 and 2]

The resin sheet (A) shown in Table 1 and the resin sheet (B) shown in Table 2 were used in the production of the carbon dioxide slow-release pack.

Here, details of the resin sheets (A) shown in Table 1 and the resin sheets (B) shown in Table 2 are as follows.

PE-1, PE-2, and PE-3 are low-density polyethylene films obtained by T-die extrusion molding, whose thicknesses are 0.01 mm, 0.04 mm and 0.08 mm, respectively.

PE-4 is a film formed of polyethylene and an inorganic filler and having fine communication holes (manufactured by Mitsui Hygiene Materials (Thailand), trade name: ESPOIR, thickness 0.019 mm).

PE-5 is high-density polyethylene nonwoven fabric having micropores (manufactured by DuPont de Nemours, Inc., trade name: TYVEK, product number: 1056-D, thickness 0.06 mm).

PP-1 is a propylene-based copolymer film (manufactured by TOYOBO CO., LTD., trade name: PYLEN FILM, product name: P1128, thickness 0.06 mm) .

PP-2 is a polypropylene-based microporous film (manufactured by 3M Japan Limited, microporous film, thickness 0.04 mm).

PMP-1 is a film including a 4-methyl-1-pentene copolymer and polybutene-1 (manufactured by RIKEN FABRO Co., product name: F.O.R. WRAP 45, thickness 0.01 mm).

PMP-2 and PMP-3 are films formed of a 4-methyl-1-pentene/propylene copolymer 1, whose thicknesses obtained by T-die extrusion molding are 0.05 mm and 0.1 mm, respectively.

PMP-4 is a film (thickness: 0.05 mm) formed of a 4-methyl-1-pentene/propylene copolymer 2 to be described later and obtained by T-die extrusion molding.

PMP-5 is a film (thickness: 0.1 mm) obtained by blending a 4-methyl-1-pentene/propylene copolymer 1 and a 4-methyl-1-pentene/propylene copolymer 2 at a blending ratio of 50/50 mass% and subjecting the mixture to T-die extrusion molding.

PMP-6 and PMP-7 are foamed sheets obtained by supercritical $CO_2$ foam extrusion sheet molding using a 4-methyl-1-pentene/propylene copolymer 1 as a raw material as in the cases of PMP-2 and PMP-3. The films have a density of 0.5 $g/cm^3$, and the thicknesses thereof are 1 mm and 0.5 mm, respectively.

PMP-8 is a sheet with a thickness of 0.6 mm obtained by T-die extrusion sheet molding using a 4-methyl-1-pentene/propylene copolymer 1 as a raw material as in the cases of PMP-2, PMP-3, PMP-6, and PMP-7.

PMP-9 is a film with a thickness of 0.1 mm obtained by T-die co-extrusion sheet molding so as to have a cross-section configuration of (L1)/(L2)/(L1)=25/50/25 $\mu$m where (L1) is a layer formed by blending a 4-methyl-1-pentene/propylene copolymer 1 and a 4-methyl-1-pentene/propylene copolymer 2 at a blending ratio of 60/40 mass%, and (L2) is a layer formed only of KRATON G1657MS manufactured by Kraton Polymers Japan Ltd. as a hydrogenated styrene-based elastomer.

PMP-10 is a sheet with a thickness of 0.26 mm obtained by thermally laminating PMP-9 sheets one by one on each of both surfaces using, as a fibrous reinforcing material, a power net structure (mesh size 3 mm, thickness 0.15 mm) produced using polyamide 6 fibers and polyurethane fibers as raw yarns.

PMP-11 is a sheet with a thickness of 0.28 mm obtained by thermally laminating PMP-9 sheets one by one on each of both surfaces using, as a fibrous reinforcing material, stretchable nonwoven fabric (mass per unit area 0.03 $kg/m^2$) produced using polypropylene fibers and polyurethane fibers.

Here, the above-described 4-methyl-1-pentene/propylene copolymer 1 is a copolymer of 4-methyl-1-pentene and propylene (content of constituent unit derived from 4-methyl-1-pentene: 72 mol%, content of constituent unit derived from propylene: 28 mol%, intrinsic viscosity [$\eta$] in decalin at 135°C: 1.5 dL/g, density measured according to ASTM D1505 (substitution method in water) : 0.84 $g/cm^3$).

The above-described 4-methyl-1-pentene/propylene copolymer 2 is a copolymer of 4-methyl-1-pentene and propylene (content of constituent unit derived from 4-methyl-1-pentene: 85 mol%, content of constituent unit derived from propylene: 15 mol%, intrinsic viscosity [$\eta$] in decalin at 135°C: 1.4 dL/g, density measured according to ASTM D1505 (substitution method in water) : 0.84 $g/cm^3$).

Next, a method of producing a carbon dioxide slow-release pack using the resin sheets (A) and (B) will be described. First, each of the resin sheets (A) and (B) was cut into a size of 100 mm $\times$ 100 mm. Next, the cut resin sheets (A) and (B) were laminated and sealed on three sides with a welding line width of 5 mm by using an impulse welding machine so as to be processed into a bag shape, and bag-shaped carbon dioxide slow-release packs were produced.

Next, carbonated water having a carbon dioxide concentration of 3,000 ppm was injected into the obtained carbon dioxide slow-release packs at a volume ratio of 50%. Next, the opening was sealed with a welding line width of 5 mm by using an impulse welding machine, and carbon dioxide slow-release packs with the carbonated water enclosed therein were obtained.

The obtained carbon dioxide slow-release packs were subjected to the evaluation of conformability and the evaluation of the blood circulation promoting property described above. The obtained results are shown in Table 3.

[Table 3]

| | Resin Sheet (A) | Resin Sheet (B) | Moisture Content of Skin [%] | | | Conformability | Blood Circulation Promoting Property | | |
| --- | --- | --- | --- | --- | --- | --- | --- | --- | --- |
| | Skin Side | Atmosphere Side | Measurement Value Immediately Before Application of Water (X) | Measurement Value Immediately After Application of Water (Y) | Increase Before and After Application of Water (Y)-(X) | | At 5 Minutes After Start | At 10 Minutes After Start | At 20 Minutes After Start |
| Example 1 | PE-2 | PE-2 | 27 | 45 | 18 | B | C | B | A |
| Example 2 | PMP-3 | PMP-3 | 31 | 48 | 17 | A | A | A | A |
| Example 3 | PMP-5 | PMP-5 | 28 | 47 | 19 | A | A | A | A |
| Example 4 | PE-1 | PE-3 | 30 | 48 | 18 | B | A | A | A |
| Example 5 | PE-2 | PE-3 | 29 | 46 | 17 | B | C | B | A |
| Example 6 | PE-4 | PE-3 | 30 | 48 | 18 | B | A | A | A |
| Example 7 | PE-5 | PE-3 | 31 | 49 | 18 | D | A | A | A |
| Example 8 | PP-2 | PP-1 | 27 | 44 | 17 | B | A | A | A |
| Example 9 | PMP-1 | PMP-8 | 30 | 45 | 15 | B | A | A | A |
| Example 10 | PMP-2 | PP-1 | 27 | 58 | 31 | A | A | A | A |
| Example 11 | PMP-3 | PMP-8 | 27 | 54 | 27 | A | A | A | A |
| Example 12 | PMP-4 | PMP-8 | 28 | 46 | 18 | A | A | A | A |
| Example 13 | PMP-6 | PP-1 | 31 | 51 | 20 | A | B | A | A |
| Comparative Example 1 | PE-3 | PE-3 | 27 | 45 | 18 | B | D | D | D |

EP 3 698 800 A1

(continued)

| | Resin Sheet (A) | Resin Sheet (B) | Moisture Content of Skin [%] | | | Conformability | Blood Circulation Promoting Property | | |
|---|---|---|---|---|---|---|---|---|---|
| | Skin Side | Atmosphere Side | Measurement Value Immediately Before Application of Water (X) | Measurement Value Immediately After Application of Water (Y) | Increase Before and After Application of Water (Y)-(X) | | At 5 Minutes After Start | At 10 Minutes After Start | At 20 Minutes After Start |
| Comparative Example 2 | PP-1 | PP-1 | 27 | 44 | 17 | D | D | D | D |
| Comparative Example 3 | PMP-7 | PP-1 | 27 | 45 | 18 | A | A | B | C |
| Reference Example 1 | PE-2 | PE-3 | 27 (value measured immediately before contact of pack to skin with no application of water) | | 0 | B | D | D | D |
| Reference Example 2 | PMP-5 | PMP-5 | 35 value measured immediately before contact of pack to skin with no application of water) | | 0 | A | D | C | C |

[Table 4]

| | Resin Sheet (A) | Resin Sheet (B) | Moisture Content of Skin [%] | | | Conformability | Blood Circulation Promoting Property | | |
|---|---|---|---|---|---|---|---|---|---|
| | Skin Side | Atmosphere Side | Measurement Value Immediately Before Application of Water (X) | Measurement Value Immediately After Application of Water (Y) | Increase Before and After Application of Water (Y) - (X) | | At 5 Minutes After Start | At 10 Minutes After Start | At 20 Minutes After Start |
| Example 14 | PMP-9 | PMP-9 | 30 | 48 | 18 | A | A | A | A |
| Example 15 | PMP-10 | PMP-10 | 29 | 47 | 18 | A | A | A | A |
| Example 16 | PMP-11 | PMP-11 | 31 | 48 | 17 | A | A | A | A |

As is obvious from Tables 3 and 4, it has been found that the carbon dioxide slow-release packs of the examples using the resin sheet (A) satisfying the requirements (A-1) and (A-2) have an excellent blood circulation promoting effect, and can maintain the blood circulation promoting effect for a long period of time.

The carbon dioxide slow-release packs of Comparative Examples 1 and 2 using the resin sheet (A) satisfying the requirement (A-2) but not satisfying the requirement (A-1) had a poor blood circulation promoting property. The carbon dioxide slow-release pack of Comparative Example 3 using the resin sheet (A) satisfying the requirement (A-1) but not satisfying the requirement (A-2) had an excellent blood circulation promoting property in an initial time, but its blood circulation promoting property was reduced with time, and the blood circulation promoting effect could not be maintained for a long period of time.

In the carbon dioxide slow-release pack using the resin sheet (A) satisfying the requirements (A-1) and (A-2), the blood circulation promoting effect was poor in a case where no moisture is applied to the skin immediately before the carbon dioxide slow-release pack is brought into contact with the skin (Reference Examples 1 and 2).

This application claims priority based on Japanese Patent Application No. 2017-203929 filed on October 20, 2017, the disclosure of which is incorporated herein in its entirety.

**Claims**

1. A carbon dioxide slow-release pack for skin which has a bag shape for slowly releasing carbon dioxide enclosed therein, thereby bringing the carbon dioxide into contact with the skin, the pack comprising:

   a resin sheet (A),
   wherein the carbon dioxide slow-release pack is formed into a bag shape by at least the resin sheet (A), and the resin sheet (A) satisfies at least the following requirements (A-1) and (A-2),
   (A-1) a carbon dioxide permeability measured under the conditions of 23°C and a relative humidity of 0% according to JIS K7126-1 is 20 L/(m$^2$·day·atm) or more, and
   (A-2) an Oken-type air permeability measured at 23°C according to JIS P8117: 2009 is less than 5 μm/(Pa·s).

2. The carbon dioxide slow-release pack for skin according to claim 1,
   wherein the resin sheet (A) further satisfies the following requirement (A-3),
   (A-3) Young's modulus E at 23°C is 10 MPa to 500 MPa.

3. The carbon dioxide slow-release pack for skin according to claim 1 or 2,
   wherein the resin sheet (A) further satisfies the following requirement (A-4),
   (A-4) tensile elongation at break at 23°C is 30% or more.

4. The carbon dioxide slow-release pack for skin according to any one of claims 1 to 3, further comprising:

   a resin sheet (B) satisfying the following requirements (B-1) and (B-2),
   (B-1) water resistance measured at 23°C according to JIS L1092A: 2009 is 350 mbar or more, and
   (B-2) an Oken-type air permeability measured at 23°C according to JIS P8117: 2009 is less than 5 μm/(Pa·s).

5. The carbon dioxide slow-release pack for skin according to claim 4,
   wherein the resin sheet (A) is disposed on a skin side, and the resin sheet (B) is disposed on an atmosphere side.

6. The carbon dioxide slow-release pack for skin according to any one of claims 1 to 5,
   wherein the resin sheet (A) includes a polyolefin film or sheet.

7. The carbon dioxide slow-release pack for skin according to claim 6,
   wherein the polyolefin film or sheet includes at least one selected from polyethylene, an ethylene-based copolymer, a propylene-based copolymer, and a 4-methyl-1-pentene-based polymer.

8. The carbon dioxide slow-release pack for skin according to claim 6 or 7,
   wherein the polyolefin film or sheet includes a microporous film or sheet.

9. The carbon dioxide slow-release pack for skin according to claim 8,
   wherein the microporous film or sheet includes one or more selected from a foamed sheet formed of a 4-methyl-1-pentene-based polymer, a polypropylene-based microporous film, and a filler-containing polyolefin-based microporous film.

10. The carbon dioxide slow-release pack for skin according to any one of claims 1 to 9,
    wherein the resin sheet (A) includes a copolymer having a constituent unit derived from 4-methyl-1-pentene and a constituent unit derived from an $\alpha$-olefin having 2 to 20 carbon atoms other than 4-methyl-1-pentene.

11. The carbon dioxide slow-release pack for skin according to any one of claims 1 to 10, further comprising:
    a fibrous reinforcing material on a surface of the resin sheet (A) or inside the resin sheet (A).

12. The carbon dioxide slow-release pack for skin according to any one of claims 1 to 11,
    wherein the resin sheet (A) includes a layer formed of a 4-methyl-1-pentene-based polymer and a layer formed of a thermoplastic elastomer.

13. The carbon dioxide slow-release pack for skin according to any one of claims 1 to 12, which is a pack for face.

14. The carbon dioxide slow-release pack for skin according to any one of claims 1 to 13,
    wherein at least one selected from carbon dioxide and a carbon dioxide source is enclosed therein.

15. A method of slowly releasing carbon dioxide to skin using the carbon dioxide slow-release pack for skin according to any one of claims 1 to 14, the method comprising:
    bringing the resin sheet (A) into close contact with the skin, and slowly releasing the carbon dioxide from the inside of the carbon dioxide slow-release pack for skin between the resin sheet (A) and the skin to continuously supply the carbon dioxide to the skin.

EP 3 698 800 A1

FIG. 1

26

# FIG. 2

HEAT SEALING REGION

OPENING (EYE PORTIONS, NOSE PORTION, AND MOUTH PORTION)

INTERNAL BUFFER MATERIAL PORTION SURROUNDED BY RESIN SHEETS (A) AND (B)

A

B

C — — — — C'

A

B

C-C' CROSS-SECTION

# FIG. 3

HEAT SEALING REGION

1   TUBE FORMED BY HEAT
    SEALING USING RESIN
    SHEET (A)

## FIG. 4

HEAT SEALING REGION

2 PORTION HAVING PILLARS IN CARBON DIOXIDE SLOW -RELEASE PACK FOR SKIN

3 PILLAR

<table>
<tr><td colspan="2" align="center">**INTERNATIONAL SEARCH REPORT**</td><td>International application No.<br>PCT/JP2018/038594</td></tr>
</table>

A. CLASSIFICATION OF SUBJECT MATTER
Int.Cl. A61K33/00(2006.01)i, A61K9/70(2006.01)i, A61K47/32(2006.01)i, A61M35/00(2006.01)i, A61P9/08(2006.01)i, A61P17/00(2006.01)i

According to International Patent Classification (IPC) or to both national classification and IPC

B. FIELDS SEARCHED

Minimum documentation searched (classification system followed by classification symbols)
Int.Cl. A61K33/00, A61K9/70, A61K47/32, A61M35/00, A61P9/08, A61P17/00

Documentation searched other than minimum documentation to the extent that such documents are included in the fields searched
Published examined utility model applications of Japan 1922-1996
Published unexamined utility model applications of Japan 1971-2019
Registered utility model specifications of Japan 1996-2019
Published registered utility model applications of Japan 1994-2019

Electronic data base consulted during the international search (name of data base and, where practicable, search terms used)
JSTPlus/JMEDPlus/JST7580 (JDreamIII)
CAplus/REGISTRY/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| X | JP 2010-222259 A (KAO CORP.) 07 October 2010, paragraphs [0001], [0007], [0026], [0029], [0035], examples 1-7, fig. 1 (Family: none) | 1-3, 6-9, 11, 13-15 |
| Y | | 4, 5 |
| Y | JP 2005-170937 A (KAO CORP.) 30 June 2005, paragraph [0017] (Family: none) | 4, 5 |

☒ Further documents are listed in the continuation of Box C. ☐ See patent family annex.

| | | |
|---|---|---|
| * | Special categories of cited documents: | |
| "A" | document defining the general state of the art which is not considered to be of particular relevance | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search<br>10 January 2019 (10.01.2019) | Date of mailing of the international search report<br>22 January 2019 (22.01.2019) |
|---|---|
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.

PCT/JP2018/038594

C (Continuation).   DOCUMENTS CONSIDERED TO BE RELEVANT

| Category* | Citation of document, with indication, where appropriate, of the relevant passages | Relevant to claim No. |
|---|---|---|
| X | JP 2005-225832 A (KAO CORP.) 25 August 2005, claim 1, paragraphs [0001], [0007], [0009]-[0012], examples 1-3 (Family: none) | 1-3, 6-9, 11, 13-15 |
| Y | | 4, 5 |
| A | JP 2002-326938 A (MITSUBISHI RAYON CO., LTD.) 15 November 2002 (Family: none) | 1-15 |
| A | WO 2011/055803 A1 (MITSUI CHEMICALS, INC.) 12 May 2011 & US 2012/0220728 A1 & EP 2497789 A1 & CN 102597027 A & KR 10-2012-0071408 A | 10, 12 |

Form PCT/ISA/210 (continuation of second sheet) (January 2015)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002326938 A
- JP 2005225832 A
- WO 0153369 A
- WO 01027124 A
- JP H03193796 A
- JP H0241303 A
- WO 2011055803 A
- JP 2017203929 A

**Non-patent literature cited in the description**

- **JIRO KADOMOTO.** blending of carbon dioxide with cosmetics and skin quality improving effect. *Fragrance Journal,* 2015, vol. 43 (7), 40-44